Europäisches Patentamt

European Patent Office

Office européen des brevets

(19)

(11) Numéro de publication: **0 099 283**
**B1**

## (12) FASCICULE DE BREVET EUROPEEN

(45) Date de publication du fascicule du brevet:
27.08.86

(51) Int. Cl.⁴: **A 61 M 16/00**

(21) Numéro de dépôt: **83401205.6**

(22) Date de dépôt: **13.06.83**

(54) Dispositif pour le traitement de l'insuffisance respiratoire d'un patient.

(30) Priorité: **13.07.82 FR 8212264**

(43) Date de publication de la demande:
**25.01.84 Bulletin 84/4**

(45) Mention de la délivrance du brevet:
**27.08.86 Bulletin 86/35**

(84) Etats contractants désignés:
**AT BE CH DE FR GB IT LI LU NL SE**

(56) Documents cités:
**DE - A - 2 809 255**
**FR - A - 2 125 449**
**FR - A - 2 309 207**
**FR - A - 2 346 025**

**BIOMEDIZINISCHE TECHNIK, vol. 19, no. 3, 1974, pages 112-117 E. APPEL: "Ein Thermistoranemometer zur widerstandsarmen Ventilationsmessung"**

(73) Titulaire: **COMPAGNIE FRANCAISE DE PRODUITS OXYGENES, 1, rue Bayard, F-75008 Paris (FR)**

(72) Inventeur: **Robert, Dominique, 10, rue des Thermes, F-69410 Champagne-au-Mont-d'Or (FR)**
Inventeur: **Perrin, François, 5, chemin du Grand Roule, F-69350 la Mulatière (FR)**
Inventeur: **Bally, Gilles, 14, cours Franklin Roosevelt, F-69006 Lyon (FR)**

(74) Mandataire: **Leclercq, Maurice et al, L'AIR LIQUIDE SOCIETE ANONYME POUR L'ETUDE ET L'EXPLOITATION DES PROCEDES GEORGES CLAUDE 75, Quai d'Orsay, F-75321 Paris Cedex 07 (FR)**

**Description**

L'invention concerne un dispositif pour le traitement de l'insuffisance respiratoire d'un patient par apport d'oxygène additionnel délivré à partir d'une source par un conduit au moins dont l'extrémité est destinée à s'insérer dans les narines du patient, ledit dispositif comportant une électro-vanne contrôlant le passage de l'oxygène additionnel, des moyens de commande de ladite vanne ainsi que des moyens de détection d'une phase d'inspiration ou d'une phase d'expiration du patient, lesdits moyens de détection engendrant, par l'intermédiaire lesdits moyens de commande, respectivement l'ouverture ou la fermeture de la vanne lors des phases d'inspiration et d'expiration du patient (voir FR-A 2 346 025).

Il existe divers appareils de ce type qui assurent au patient un enrichissement en oxygène de l'air normalement inspiré par les voies respiratoires habituelles, c'est-à-dire essentiellement les narines ou encore la bouche. L'un de ces appareils les plus répandus est la lunette d'oxygénothérapie qui peut s'adapter sur le visage et comporte deux appendices permettant un apport d'oxygène au deux narines du patient.

Toutefois, les appareils actuellement connus sont munis d'une simple vanne manuelle insérée généralement dans le conduit précité et que le patient ouvre lorsque l'appareil est en service. Ce système a toutefois l'inconvénient que l'oxygène est délivré de façon continue alors que son apport n'est utile que pendant les phases inspiratoires et se fait en pure perte pendant les phases expiratoires.

Cette perte, pécuniairement dommageable en soi, constitue un inconvénient encore plus important dans le cas des appareils portatifs pour lesquels la capacité de la source l'oxygène détermine le rayon d'action.

Il est connu du brevet français FR-A 2 346 025 d'asservir l'alimentation en gaz de respiration aux phases d'inspiration et d'expiration par ouverture d'une vanne permettant le passage dudit gaz lors de l'inspiration et fermeture de celle-ci lors de l'expiration. La détection des phases d'inspiration et d'expiration se fait à l'aide d'un capteur de pression qui commande l'ouverture ou la fermeture d'une vanne reliant le circuit d'alimentation du patient à un réservoir intermédiaire.

Le brevet allemand DE-A 2 809 255 décrit un nébuliseur permettant d'injecter par insufflation une dose d'une substance de diagnostic à un patient.

L'appareil comporte dans ce but une thermistance dont le refroidissement, lors de l'inspiration par le patient déclenche un compteur qui ouvre une vanne d'air comprimé pendant un intervalle de temps prédéterminé, cet air comprimé provoquant l'éjection d'une dose de médicaments.

Pour assurer le déclenchement du compteur à l'aide de la thermistance, celle-ci est polarisée à l'aide d'un courant suffisant pour qu'aucune variation de sa résistance ne se produise à la température de l'air expiré par le patient.

L'inspiration de l'air par le patient provoque le refroidissement de la thermistance, ce qui engendre une augmentation de sa résistance, donc de la tension développée à ses bornes et le démarrage du compteur.

Compte tenu de cet art antérieur, le problème posé est l'utilisation d'une thermistance en tant que capteur de température, de manière à assurer grâce à celle-ci le déclenchement de l'injection du gaz de respiration par l'inspiration du patient et l'arrêt de cette injection par l'expiration du patient.

La présente invention a pour but de résoudre le problème ainsi posé et propose à cet effet un dispositif caractérisé en ce que lesdits moyens de détection sont constitués par une thermistance accolée à l'extrémité du conduit, ladite thermistance étant connectée en série avec une source de tension et une résistance, et en ce que lesdits moyens de commande comportent un dérivateur dont l'entrée est reliée à la thermistance et dont la sortie est connectée à un organe de commande de l'électro-vanne, les variations de tension aux bornes de la thermistance, résultant d'une variation de température de la thermistance due à une phase d'inspiration ou d'expiration du patient, créant un signal électrique à la sortie du dérivateur qui engendre l'ouverture ou la fermeture de ladite vanne respectivement au cours des phases d'inspiration ou d'expiration du patient.

Le dispositif selon l'invention permet donc, grâce à l'organe thermosensible, de détecter les phases inspiratoires et expiratoires et de commander en conséquence l'ouverture ou la fermeture de l'électro-vanne, donc l'alimentation en oxygène. Bien entendu, le réglage des composants du dispositif est tel que l'électro-vanne soit ouverte pendant les phases inspiratoires et fermée pendant les phases expiratoires, assurant ainsi un apport d'oxygène aux seuls instants utiles.

L'économie d'oxygène qui en résulte entraîne non seulement une diminution des frais puisqu'elle permet d'espacer la recharge des bouteilles, mais elle permet également, dans le cas d'appareils portatifs, une autonomie notablement plus importante qu'avec les appareils classiques.

Selon une autre caractéristique de l'invention, l'organe de commande provoque l'ouverture de l'électro-vanne, fermée en l'absence de tension, pendant les phases inspiratoires.

Selon une autre caractéristique de l'invention, l'organe de commande provoque la fermeture de l'électro-vanne, ouverte en l'absence de tension, pendant les phases expiratoires.

Selon encore une autre caractéristique de l'invention appliquée à une lunette d'oxygénothérapie dans laquelle le conduit d'oxygène précité comporte deux appendices destinés à s'insérer dans les narines du patient, l'organe thermosensible est constitué par deux thermistances insérées en parallèle dans le circuit d'alimentation en courant électrique, lesdites thermistances étant accollées aux appendices précités.

D'autres caractéristiques et avantages de l'invention apparaîtront au cours de la description qui va suivre.

Dans les dessins annexés et donnés uniquement à titre d'exemple:

– la figure 1 représente le schéma d'un dispositif selon l'invention;
– la figure 2 est une vue partielle du schéma de la figure 1 montrant une variante de réalisation de l'invention;
– la figure 3 représente une lunette d'oxygénothérapie munie du dispositif selon l'invention.

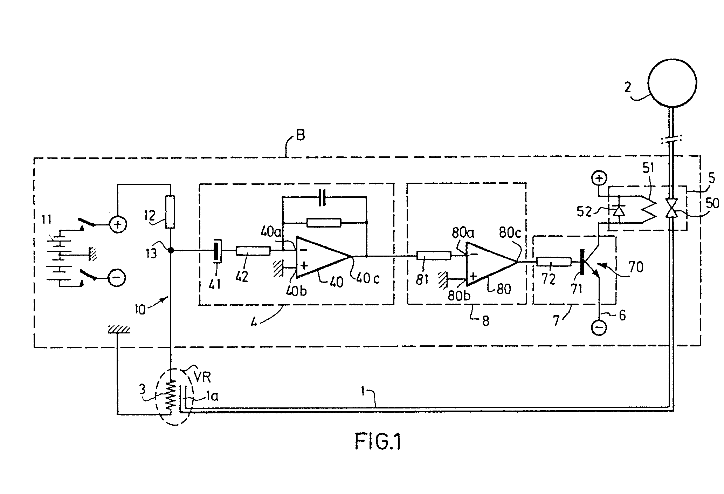

En se référant à la figure 1, on voit qu'un conduit 1 est relié, par l'intermédiaire d'un détecteur non représenté, à une source d'oxygène d'appoint sous pression 2 et comporte une extrémité ouverte 1a qui débouche au voisinage immédiat des voies respiratoires du patient indiquées schématiquement en VR.

Un organe thermosensible constitué par une thermistance 3 est également placé au voisinage immédiat des voies respiratoires VR et de l'extrémité 1a du conduit 1. Cette thermistance 3 est insérée dans un circuit de détection de variations de température 10 comportant une source de tension 11 et une résistance 12 en série avec la thermistance 3, cette résistance et cette thermistance ayant l'une de leurs bornes reliée à un point commun 13.

Les variations de températures entre l'air inspiré et l'air expiré par le patient entraînent des variations correspondantes de la valeur de la résistivité de la thermistance 3 alors que la résistance est insensible à ces variations de température. Il apparaît donc, aux bornes de la thermistance, des variations de tension correspondant à ces variations de température et par conséquent, un signal de tension entre la borne 13 et la masse.

Le dispositif comporte également un dérivateur 4 qui reçoit les signaux de tension précités et délivre des signaux de sortie correspondants, une électro-vanne 5 qui, en l'absence de tension, est normalement fermée et qui est montée dans le conduit 1, un circuit d'alimentation électrique 6 de ladite électro-vanne, un organe de commande 7 dudit circuit d'alimentation, ce circuit 7 recevant signaux du dérivateur 4 par l'intermédiaire d'un organe de mise en forme 8. L'ensemble du circuit 10 (sauf la thermistance), du dérivateur 4, de l'électro-vanne 5, du circuit 6, de l'organe 7 et de l'organe de mise en forme 8 sont logés dans un boîtier indiqué en B.

Le dérivateur 4 comprend un amplificateur 40 dont l'une des bornes 40a est reliée, par l'intermédiaire d'une capacité de liaison 41 et d'une résistance d'entrée 42, à la borne 13 du circuit 10 tandis que son autre borne d'entrée 40b est reliée à la masse.

L'électro-vanne 5 comporte un élément 50 d'ouverture et de fermeture du conduit 1 et un enroulement 51 inséré dans le circuit d'alimentation 6. Une diode 52 assure la protection du transistor 70.

L'organe de commande 7 du circuit d'alimentation 6 est constitué essentiellement par un transistor 70 comportant une base 71 et muni d'une résistance d'entrée 72, cette base 71 étant polarisée par les signaux apparaissant à la sortie 40c de l'amplificateur 40, ceux-ci étant appliqués à la résistance 72 par l'intermédiaire de l'organe de mise en forme 8. Ce dernier comporte essentiellement un amplificateur 80 dont l'une des bornes 80a (négative) est reliée, par l'intermédiaire d'une résistance d'entrée 81, à la borne 40c, son autre borne d'entrée 80b (positive) étant reliée à la masse tandis que sa borne de sortie 80c est reliée directement à la résistance 72.

Selon les phases d'inspiration et d'expiration du patient, la thermistance 3 est soumise à des variations de température qui font apparaître, à la borne 13, un signal de tension variable. Ce signal est amplifié et dérivé par le dérivateur 4 et transformé par conséquent en un signal positif ou négatif qui est appliqué, par l'intermédiaire de l'organe de mise en forme 8, à la base 71 du transistor. Pendant la phase inspiratoire, le signal de sortie de l'organe de mise en forme 8 est positif et commande l'ouverture de l'électro-vanne tandis que pendant la phase expiratoire, il est négatif et commande sa fermeture.

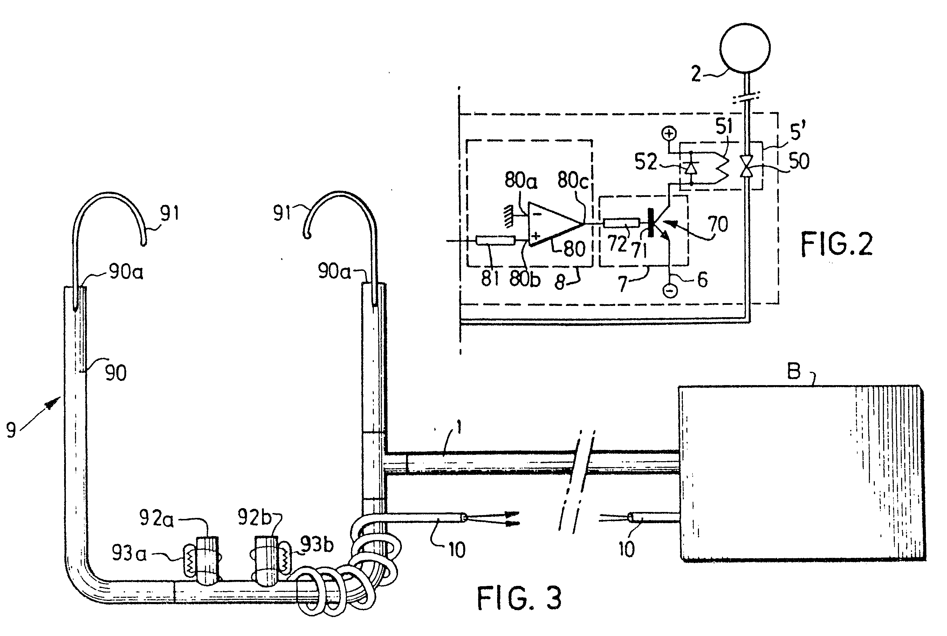

Dans la variante de réalisation représentée à la figure 2 dans laquelle les mêmes chiffres de référence désignent les mêmes éléments que dans la fig. 1, la résistance 81 est reliée à la borne positive 80b de l'amplificateur 80 dont la borne négative 80a est reliée à la masse. Par ailleurs, l'électro-vanne 5' montée dans le circuit 1 est normalement ouverte en l'absence de tension.

Pendant la phase inspiratoire, le signal de sortie de l'organe de mise en forme 8 est négatif et commande ou maintient l'ouverture de l'électro-vanne 5', tandis que pendant la phase expiratoire il est positif et commande sa fermeture. Ce mode de montage a l'avantage, en cas de déficience de l'alimentation électrique ou d'un élément au système électronique, de permettre l'arrivée d'oxygène aux voies respiratoires du patient.

On a représenté à la figure 3, dans laquelle les mêmes chiffres de référence désignent les mêmes éléments que dans les figures 1 et 2, une application de l'invention à une lunette d'oxygénothérapie. La lunette, désignée d'une façon générale par 9, est formée d'une tubulure 90 fermée à ses extrémités 90a et épousant sensiblement la forme du visage de façon à pouvoir être portée par un patient à la manière de lunettes optiques. Des branches 91, aux extrémités de la tubulure 90 permettent de fixer la lunette sur les oreilles. La tubulure 90 comporte deux appendices 92a et 92b dont les extrémités sont ouvertes et qui débouchent, lorsque la lunette est en place sur le visage, en face des narines du patient qui reçoit ainsi l'oxygène d'apport amené par le conduit 1. Sur chacun des appendices 92a et 92b est montée une thermistance 93a et 93b, ces deux thermistances étant montées en parallèle et insérées dans le circuit 10 de détection de variation de température (voir la figure 1). Le fait de prévoir deux thermistances en parallèle permet d'augmenter la sécurité de l'appareil, celui-ci continuant à fonctionner même en cas de défaillance de l'une des thermistances.

L'invention n'est nullement limitée au mode de réalisation décrit et représenté qui n'a été donné qu'à titre d'exemple non limitatif.

**Revendications**

1. Dispositif pour le traitement de l'insuffisance respiratoire d'un patient par apport d'oxygène additionnel délivré à partir d'une source (2) par un conduit (1) au moins dont l'extrémité (1a) est destinée à s'insérer dans les narines du patient, ledit dispositif comportant une électro-vanne (5, 5') contrôlant le passage de l'oxygène additionnel, des moyens de commande (4, 7, 8) de ladite vanne (5, 5') ainsi que des moyens de détection d'une phase d'inspiration ou d'une phase d'expiration du patient, lesdits moyens de détection engendrant, par l'intermédiaire desdits moyens de commande, respectivement l'ouverture ou la fermeture de la vanne (5, 5') lors des phases d'inspiration et d'expiration du patient, caractérisé en ce que lesdits moyens de détection sont constitués par une thermistance (3) accolée à l'extrémité (1a) du conduit (1), ladite thermistance (3) étant connectée en série avec une source de tension (11) et une résistance (12), et en ce que lesdits moyens de commande comportent un dérivateur (4) dont l'entrée est reliée à la thermistance (3) et dont la sortie est connectée à un organe de commande (7) de l'électro-vanne (5, 5'), les variations de tension aux bornes de la thermistance (3), résultant d'une variation de température de la thermistance due à une phase d'inspiration ou d'expiration du patient, créant un signal électrique à la sortie du dérivateur (4) qui engendre l'ouverture ou la fermeture de ladite vanne (5, 5') respectivement au cours des phases d'inspiration ou d'expiration du patient.

2. Dispositif pour le traitement de l'insuffisance respiratoire d'un patient, selon la revendication 1 formant une lunette d'oxygénothérapie (9) dans laquelle le conduit d'oxygène (1) précité communique avec deux appendices (92a, 92b) destinés à s'insérer dans les narines du patient, caractérisé en ce qu'il comporte deux thermistances (93a, 93b) insérées en parallèle dans le circuit de détection des variations de température (10), lesdites thermistances (93a, 93b) étant accolées aux appendices (92a, 92b) précités.

**Patentansprüche**

1. Vorrichtung zum Behandeln von Lungeninsuffizienz eines Patienten durch Zufuhr von zusätzlichem Sauerstoff, der von einer Quelle (2) durch mindestens eine Leitung (1) zugeführt wird, dessen Ende (1a) dazu bestimmt ist, in die Nasenlöcher des Patienten eingefügt zu werden, wobei die Vorrichtung ein Elektroventil (5, 5'), welches den Durchgang des zusätzlichen Sauerstoffes steuert, Steuermittel (4, 7, 8) des Ventiles (5, 5') sowie Mittel zum Erfassen einer Einatmungs- oder einer Ausatmungsphase des Patienten aufweist, wobei die Mittel zum Erfassen durch die Steuermittel jeweils das Öffnen oder das Schliessen des Ventils (5, 5') während der Einatmungs- und Ausatmungsphasen des Patienten erzeugen, dadurch gekennzeichnet, dass die Mittel zum Erfassen durch einen Thermistor (3) gebildet sind, der mit dem Ende (1a) der Leitung (1) verbunden ist, wobei der Thermistor (3) in Reihe mit einer Spannungsquelle (11) und einem Widerstand (12) verbunden ist, und dass die Steuermittel ein Differenzierglied (4) aufweisen, dessen Eingang mit dem Thermistor (3) verbunden ist und dessen Ausgang mit einem Steuerorgan (7) des Elektroventils (5, 5') verbunden ist, wobei die Spannungsveränderungen an den Klemmen des Thermistors (3), welche eine Temperaturveränderung des Thermistors infolge einer Einatmungs- oder Ausatmungsphase des Patienten hervorruft, ein elektrisches Signal am Ausgang des Differenziergliedes 4 erzeugt, welches das Öffnen oder das Schliessen des Ventiles (5, 5') jeweils im Verlaufe der Einatmungs- oder Ausatmungsphasen des Patienten erzeugt.

2. Vorrichtung zum Behandeln von Lungeninsuffizienz eines Patienten nach Anspruch 1 unter Bildung einer Sauerstofftherapiebrille (9), in welcher die Sauerstoffleitung (1) mit zwei Ansätzen (92a, 92b) verbunden ist, die dazu bestimmt sind, in die Nasenlöcher des Patienten eingefügt zu werden, dadurch gekennzeichnet, dass sie zwei Thermistoren (93a, 93b) aufweist, die parallel im Kreis zum Erfassen von Temperaturveränderungen (10) eingefügt sind, wobei die Thermistoren (93a, 93b) mit den genannten Ansätzen (92a, 92b) verbunden sind.

**Claims**

1. Device for treatment of the respiratory insufficiency of a patient by contribution of supplementory oxygen delivered from a source (2) by at least one conduit (1) the end (1a) of which is defined to insert this device into the patient's nostrils, the device comprising an electro valve (5, 5') controlling the passage of the supplementory oxygen, commanding means (4, 7, 8) of the said valve (5, 5') as well as means to detect an inspiration phase or an expiration phase of the patient, the said detection means creating by means of the said commanding means each the opening or the closing of the valve (5, 5') during the inspiration phase and expiration phase of the patient, characterized in that the said detection means are constituted by a thermistor (3) joined to the end (1a) of the conduit (1), the said thermistor (3) being connected in series with a voltage source (11) and a resistance (12), and in that the said commanding means comprise a derivator (4) the input of which is connected to the thermistor (3) and the output of which is connected to a commanding element (7) of the electro valve (5, 5') the voltage variations at the terminals of the thermistor (3), resulting from a temperature variation of the thermistor due to an inspiration phase or an expiration phase of the patient, creating an electrical signal at the output of the derivator (4), which signal causes the opening or the closing of the said valve (5, 5') respectively in the course of the inspiration phase or expiration phase of the patient.

2. Device for treating the respiratory insufficiency of a patient according to claim 1 forming an oxygenous therapeutical spectacle (9) and support, respectively, within which the oxygen conduit (1) communicates whith two extensions (92a, 92b) defined to be inserted in the nostrils of the patient, characterized in that it comprises two thermistors (93a, 93b) inserted in parallel in the circuit (10) for detecting the temperature variations, the said thermistors (93a, 93b) being connected to the said extensions (92a, 92b).

FIG.1

FIG.2

FIG. 3